# EUROPEAN PATENT APPLICATION

(11) **EP 4 772 124 A1**
(43) Date of publication of application: **08.07.2026**
(21) Application number: 25217866.0
(22) Date of filing: 24.11.2025
(51) Int. Cl.: A61B 18/14

(54) **MEDICAL PROBE INCLUDING LEAVES WITH FREE DISTAL ENDS**

(30) Priority: 31.12.2024 US 202419007102
(71) Applicant: Biosense Webster (Israel) Ltd., 2066717 Yokneam (IL)
(72) Inventor: VAN NIEKERK, Pieter Emmelius, Irvine, 92618 (US); HIGHSMITH, Debby, Irvine, 92618 (US); BASU, Shubhayu, Irvine, 92618 (US); SUAREZ, Paul, Irvine, 92618 (US); HENRIQUEZ, Jamie Lynn, Irvine, 92618 (US); BAR-TAL, Meir, 2066717 Yokneam (IL); BERGER, Abraham, 2066717 Yokneam (IL); BERGER, Omer, 2066717 Yokneam (IL)
(74) Representative: Carpmaels & Ransford LLP

(57) **Abstract**

The disclosed technology includes an end effector of a medical probe. The end effector includes spines and a membrane. The spines are coupled to a distal portion of a tubular member, with each spine including a proximal spine end coupled to the distal portion of the tubular member and a free distal end. The membrane and spines are fixed relative to each other to define a single member. The membrane defines a plurality of leaves, each of which, in an expanded configuration, extending curvilinearly outward and being separated from adjacent free distal ends of adjacent leaves of the plurality of leaves. Each leaf includes flexible circuits including electrodes.

## Description

### FIELD

The present technology relates generally to medical devices, and in particular medical probes with electrodes, and further relates to, but not exclusively, medical probes suitable for use to map or ablate tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0196372A1, 2021/0177503A1, and 2021/0186604A1, and U.S. Pat. No. 11,540,877 each of which are incorporated herein by reference in their entireties and attached in the Appendix hereto.

Regions of cardiac tissue can be mapped by a catheter to identify the abnormal electrical signals. Some catheter ablation procedures especially those with persistent atrial fibrillation may be performed using electrophysiology (EP) mapping to target areas of aberrant electrical signals. Such EP mapping may include the use of diagnostic electrodes configured to monitor electrical signals within the cardiovascular system to pinpoint the location of aberrant conductive tissue sites that are responsible for the arrhythmia. Examples of an EP mapping system are described in U.S. Patent No. 5,738,096, incorporated herein in its entirety by reference. Examples of EP mapping catheters are described in U.S. Patent No. 9,907,480, U.S. Patent Pub. No. 2018/0036078, and U.S. Patent Pub. No. 2018/0056038, each of which are incorporated herein by reference in their entireties.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, Calif.

In current practice, the effectiveness of the delivery of IRE energy is dependent on the skill of the physician, meaning the patient can suffer from incomplete isolation of target areas. In order to effectively deliver the IRE energy to ablate, ablation catheters typically need to be reoriented multiple times during a procedure, which increases procedure time as well as complicates the ablation process. Moreover, existing catheters generally require stiff internal structural members to ensure that a predetermined configuration is maintained. The stiffness is a disadvantage during manipulation in the body organ as it can prevent electrodes from contacting the tissue. Other catheters can include flexible end effectors designed to overcome this disadvantage. These catheters can include layered components that can be time-consuming, complex, and expensive to manufacture and assemble. Accordingly, there is a need for an improved end effector of a medical probe that addresses these problems that is capable of ablating and mapping.

### SUMMARY

There is provided, in accordance with the disclosed technology, an end effector of a medical probe, the medical probe comprising a tubular member extending along a longitudinal axis and including a distal portion and a proximal portion. The end effector comprises: a plurality of spines coupled to the distal portion with each spine including a proximal spine end coupled to the distal portion of the tubular member and a free distal end; a membrane connected to the respective plurality of spines so that the membrane and the plurality of spines are fixed relative to each other to define a single member, the membrane defining a plurality of leaves, each leaf including a proximal end configured to be connected to a distal end of the tubular member and a free distal end that, in an expanded configuration, extends curvilinearly outward from the longitudinal axis and is separated from adjacent free distal ends of adjacent leaves of the plurality of leaves. Each leaf comprises: a first flexible circuit disposed on the membrane on a first side of the spine, the first flexible circuit comprising a first electrode; and a second flexible circuit disposed on the membrane on a second side of the respective spine, the second flexible circuit comprising a second electrode.

There is further provided, in accordance with the disclosed technology, a medical system comprising: a medical probe comprising an elongated tubular member and an end effector connected to a distal end of the elongated probe body, the tubular member and the end effector extending along a longitudinal axis; and an ablation energy generator. The end effector comprises a plurality of leaves, each leaf including a proximal end connected to a distal end of the tubular member and a free distal end that, in an expanded configuration, extends curvilinearly outward from a longitudinal axis of the end effector and is separated from adjacent free distal ends of adjacent leaves of the plurality of leaves. Each leaf comprises: a spine with a free distal end; a membrane connected to the respective spine so that the spine is fixed relative to the membrane; a first flexible circuit disposed on the membrane on a first side of the spine; a second flexible circuit disposed on the membrane on a second side of the respective spine, the second flexible circuit comprising a second electrode; a first ablation electrode disposed on the first flexible circuit; and a second ablation electrode disposed on either the first flexible circuit or the second flexible circuit. The ablation generator is configured to provide ablation pulses between the first ablation electrode and the second ablation electrode of each leaf.
There is further provided, in accordance with the disclosed technology, a method of manufacturing an end effector for a medical probe. The method comprises: forming a plurality of spines each comprising a free distal end, the free distal ends extending away from a longitudinal axis; disposing first flexible circuits on a first side of each spine of the plurality of spines, each first flexible circuit comprising a first flexible substrate and a first electrode; disposing second flexible circuits on a second side of each spine of the plurality of spines, each second flexible circuit comprising a second flexible substrate and a second electrode; placing a first sheet of insulative material in contact with the first flexible circuits; placing a second sheet of insulative material in contact with the second flexible circuits; molding the first sheet and the second sheet to envelop the first flexible circuits and the second flexible circuits; and defining voids in the first and the second sheet such that the first sheet, the second sheet, the first flexible circuits, the second flexible circuits, and the spines collectively form a plurality of leaves, the leaves comprising free distal ends that are separated from one another.

### BRIEF DESCRIPTION OF THE DRAWINGS

FIG. 1 is a schematic pictorial illustration of a medical system including a medical device that includes a medical probe with a distal tip with leaves and electrodes, in accordance with the disclosed technology;
FIG. 2 is a schematic pictorial illustration showing a perspective view of a distal side of a distal tip of the medical probe in an expanded configuration, in accordance with the disclosed technology;
FIG. 3 is a schematic pictorial illustration showing a perspective view of a proximal side of a distal tip of the medical probe in an expanded configuration, in accordance with the disclosed technology;
FIG. 4A is a schematic pictorial illustration showing a cross-sectional view, taken along line 4A-4A in FIG. 2, of the distal tip, in accordance with the disclosed technology;
FIG. 4B is a schematic pictorial illustration showing a cross-sectional view, taken along line 4B-4B in FIG. 2, of the distal tip, in accordance with the disclosed technology;
FIG. 5A is a schematic pictorial illustration showing the cross-sectional view of FIG. 4A, showing an exemplary ablation configuration of the electrodes, in accordance with the disclosed technology;
FIG. 5B is a schematic pictorial illustration showing a similar cross-sectional view as that of FIG. 5A, showing an alternative ablation configuration of the electrodes, in accordance with the disclosed technology;
FIG. 5C is a schematic pictorial illustration showing a similar cross-sectional view as that of FIG. 5A, showing an alternative ablation configuration of the electrodes, in accordance with the disclosed technology;
FIG. 6A is a schematic pictorial illustration showing a similar cross-sectional view of FIG. 5A, showing an alternative configuration of the electrodes, in accordance with the disclosed technology;
FIG. 6B is a schematic pictorial illustration showing a similar cross-sectional view of FIG. 5B, showing an alternative configuration of the leaves, in accordance with the disclosed technology;
FIG. 7 is a schematic pictorial illustration showing a similar cross-sectional view as that of FIG. 5A, showing an alternative configuration of the leaves, in accordance with the disclosed technology;
FIG. 8 is a schematic pictorial illustration showing a similar cross-sectional view as that of FIG. 5A, showing an alternative configuration of the leaves, in accordance with the disclosed technology;
FIG. 9 is a schematic pictorial illustration showing a similar cross-sectional view as that of FIG. 5A, showing an alternative configuration of the leaves, in accordance with the disclosed technology;
FIG. 10 is a flow chart depicting a method of manufacturing a distal tip of a medical probe, in accordance with the disclosed technology;
FIG. 11 is a schematic pictorial illustration showing a first example electrode configuration for the example distal tip including elongated ablation electrodes and diagnostic electrodes positioned alongside the ablation electrodes, in accordance with the disclosed technology;
FIG. 12 is a schematic pictorial illustration showing a second example electrode configuration for the example distal tip including elongated ablation electrodes which are respectively segmented into small parallel stripes and diagnostic electrodes positioned alongside the ablation electrodes, in accordance with the disclosed technology;
FIG. 13 is a schematic pictorial illustration showing a third example electrode configuration for the example distal tip including rectangular elongated ablation electrodes without diagnostic electrodes, in accordance with the disclosed technology; and
FIG. 14 is a schematic pictorial illustration showing a fourth example electrode configuration for the example distal tip including rectangular elongated ablation electrodes interrupted by diagnostic electrodes, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Each phase of the biphasic and monophasic pulse preferably has a square shape having an essentially constant voltage amplitude during a majority of the phase duration. Phases of the biphasic pulse may be separated in time by an interphase delay.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

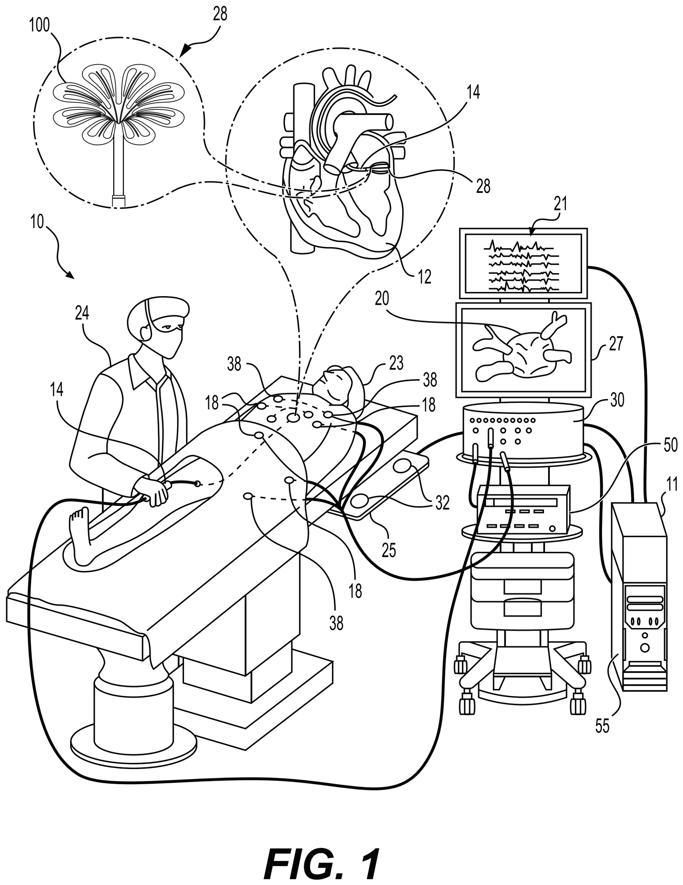

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating or catheters dedicated for both sensing and ablating. An example catheter/medical probe 14 that is configured for sensing IEGM is illustrated herein. Physician 24 brings a catheter shaft with a distal tip 28 (*e.g.,* a multi-layered end effector 100) of catheter 14 into contact with the heart wall for sensing a target site in heart 12. For ablation, physician 24 would similarly bring a distal end of an ablation catheter to a target site for ablating.

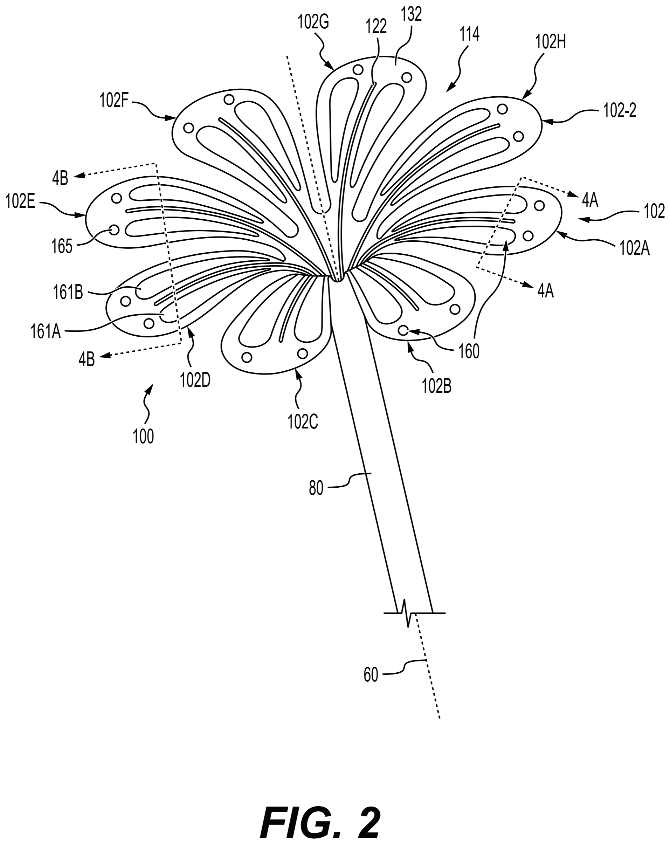

Catheter 14 is an exemplary catheter that includes multiple electrodes 160 (see FIG. 2), which include ablation electrodes 161 and diagnostic electrodes 165. The diagnostic electrodes 165 are positioned proximal to or are surrounded by the ablation electrodes 161 and are configured to sense the IEGM signals and to aid in confirming contact with tissue. In examples described herein, electrodes 160 can be configured to deliver ablation energy (IRE or RF) to tissue in heart 12. In addition to using electrodes 160 to deliver ablation energy, the electrodes 160 can also be used to determine the location of the end effector 100 or to measure a physiological property such as local surface electrical potentials at respective locations on tissue in heart 12. The electrodes 160 can be biased such that a greater portion of the electrode 160 faces outwardly from the end effector 100 such that the electrodes 160 deliver a greater amount of electrical energy outwardly away from the end effector 100 (i.e., toward the heart 12 tissue) than inwardly toward the end effector 100.

Examples of materials ideally suited for forming electrodes 160 (which include electrodes 161 and 165) include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes 160 on the inner sides of the spines), and then to the blood pool in heart 12. Additionally, silver epoxy/inks can be employed, which can be used to increase surface area to reduce impedance and improve flexibility. In some examples, impedance reducing coatings, such as iridium oxide (IrOx) or a platinum-iridium (PtIr) alloy can be employed.

Catheter 14 may additionally include a position sensor embedded in or near distal tip 28 for tracking position and orientation of distal tip 28. Optionally and preferably, the position sensor is a magnetic based position sensor including three magnetic coils for sensing three-dimensional (3D) position and orientation.

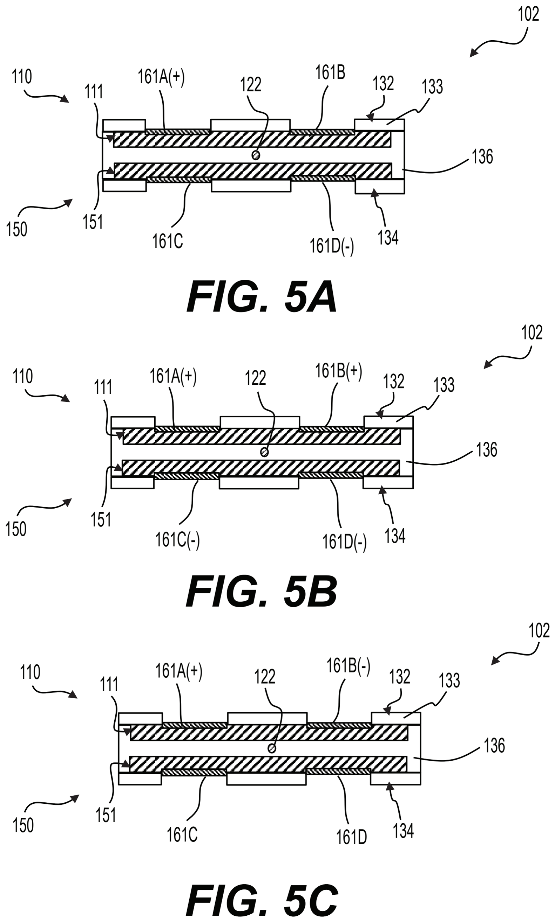

Magnetic based position sensor may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference. The end effector 100 may further include one or more inductive coils configured to provide electrical signals to the magnetic based position sensing system to determine location or orientation of the end effector. For instance, the end effector 100 may include inductive loops or coils similar to as illustrated in Figures 5A and 5B of U.S. Patent Publication No. 2024/0215894 incorporated by reference in its entirety herein and attached in the Appendix hereto. In some examples, one or more inductive coils in the end effector 100 may be used together with the position sensor to determine location or orientation of the end effector 100.

System 10 includes one or more electrode patches 38 positioned for skin contact on patient 23 to establish location reference for location pad 25 as well as impedance-based tracking of electrodes 160. For impedance-based tracking, electrical current is directed toward electrodes 160 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 160 of catheter 14. Recorder 11 may include pacing capability for pacing the heart rhythm or may be electrically connected to a standalone pacer.

System 10 may include an ablation energy generator 50 that is adapted to conduct ablative energy to one or more of electrodes 160 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage direct current (DC) or alternating current (AC) pulses as may be used to effect irreversible electroporation (IRE), or combinations thereof. The ablation energy generator 50 is preferably configured to provide biphasic bipolar pulses to induce IRE while keeping tissue temperature below thermal ablation temperatures. Additionally, or alternatively the ablation generator 50 is configured to provide monophasic IRE pulses, unipolar IRE pulses, thermal ablation electrical signals, or combinations thereof. For instance, the ablation energy generator 50 can be configured to provide pulses similar to as described in in U.S. Patent Pub. No. 2021/0169550A1, 2021/0177503A1, 2021/0186604A1, and 2023/0009191A1 and U.S. Patent No. 11,540,877B2, each of which are incorporated herein by reference in their entireties and attached in the Appendix hereto. U.S. Patent No. 11,540,877B2 corresponds to U.S. Patent Pub. No. 2021/0161592A1, which is incorporated here by reference in its entirety.

For instance, as described in U.S. Patent No. 11,540,877B2, the generator 50 can be configured to apply bipolar pulses having an amplitude sufficient to cause IRE in the tissue contacted by the electrodes and also RF energy having power sufficient to thermally ablate the tissue contacted by the electrodes. In some embodiments, the sequence of bipolar pulses includes pulses having an amplitude of at least 200 V, and a duration of each of the bipolar pulses is less than 20 µs. Additionally, or alternatively, the RF signal has a frequency between 350 and 500 kHz and an amplitude between 10 and 200 V. The end effector may further include temperature sensors and the electrical signal generator can be configured to apply the signals responsively to a temperature measured by the temperature sensors. In some embodiments, the IRE signal may have parameters as indicated in Table 1 of U.S. Patent No. 11,540,877B2. Note that the "bipolar pulse" as described in U.S. Patent No. 11,540,877B2 is referred as a "biphasic pulse" herein which relates to the shape of an electrical signal; whereas a "bipolar pulse" as described herein relates to the arrangement of electrodes receiving the pulse as defined herein above.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations. The PIU 30 can control the generator 50 to provide electrical energy to the ablation electrodes of the end effector 100 according to the ablation protocols described above and in the above incorporated references. The PIU 30, workstation 55, and the generator 50 can collectively be considered an ablation system console having one or more output ports configured to provide ablation energy to the ablation electrodes, one or more processors, and non-transitory computer-readable medium in communication with the processor to cause the ablation system console to provide ablation energy as described above and in the examples herein below.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

In some examples, the system 10 further includes an irrigation system configured to irrigate during IRE. In some embodiments, the PIU 30 is configured to control the irrigation system to provide irrigation to the catheter end effector similar to as described in U.S. Patent Pub. No. 2021/0196372A1 incorporated by reference in its entirety herein and attached in the Appendix hereto. The irrigation fluid may exit the distal end of the catheter 14 through a port at the distal end of the shaft 80 or through pores in the body of the end effector 100.

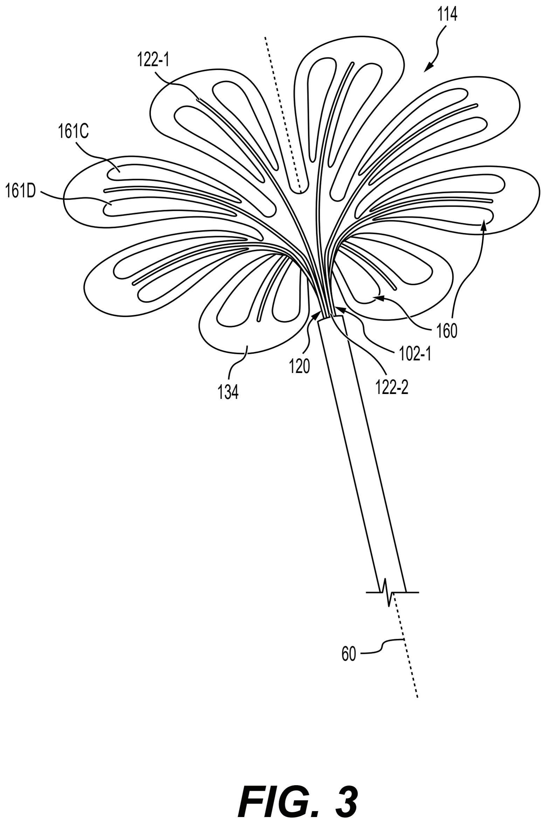

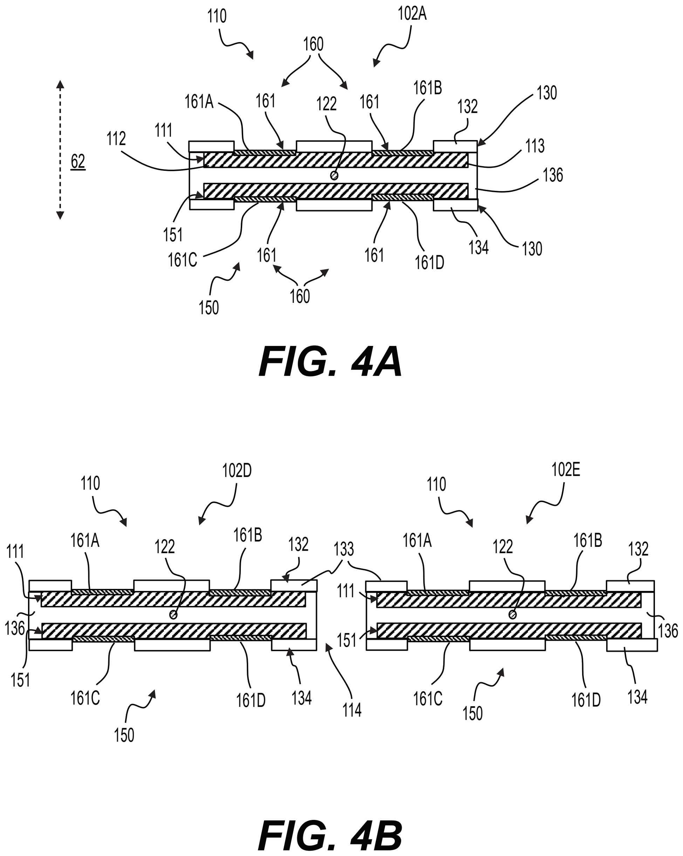

FIG. 2 is a schematic pictorial illustration showing a perspective view of a distal side of a distal tip 28 of the catheter 14. FIG. 3 is a schematic pictorial illustration showing a perspective view of a proximal side of the distal tip 28 of the catheter 14. FIG. 4A is a schematic pictorial illustration of a cross-sectional view of a portion of the distal tip 28.

It is noted that FIG. 4A, as well as the other cross-sectional figures in the present application, are depicted with the illustrated portion of the end effector 100 being flat/planar. However, those skilled in the art will appreciate that these illustrations are merely intended to relay, to the reader, potential configurations of the electrodes 160 or layers of the end effector 100. In use, the end effector has a shaped similar to a flower with petals (referred to herein as leaves); therefore, a more technically accurate depiction of cross-sectional views of the end effector 100 could be arcuate or otherwise not completely planar.

Making reference now to FIGs. 2-3, a distal tip 28 (FIG. 1) of the catheter/medical probe 14 includes an end effector 100 extending from an elongated probe shaft 80 and along a longitudinal axis 60. In some examples, the probe shaft 80 includes a tubular member 80 that extends along the longitudinal axis 60 and carries the aforementioned irrigation system. The tubular member 80 includes a distal portion and a proximal portion connected to the PIU 30. The end effector 100 is connected to the distal portion of the tubular member 80 includes a spine framework 120 and a three-dimensional structure membrane 130.

The framework 120 includes a plurality of spines 122 extending radially outwardly from the longitudinal axis 60. In the example of FIGs. 2-3, each spine 122 has a free distal end 122-1 and a fixed proximal end 122-2 that is connected/coupled to the tubular member 80. In an expanded configuration (FIGs. 2-3), each spine 122 curves outwardly from the longitudinal axis 60 from the fixed proximal end 122-2 to the free distal end 122-1. In the present example, eight spines 122 are employed that are angularly/circumferentially disposed about the longitudinal axis 60. However, any appropriate number of spines 122 (e.g., at least three, four, five, etc.) can be employed without departing from the spirit and scope of the present disclosure, provided that they are capable of supporting the three-dimensional structure membrane 130 (discussed in greater detail below).

In some examples, the framework 120 is unitary (i.e., a monolithic structure). In such examples, the framework 120 can be formed from a planar or cylindrical tube stock of material using any suitable method. For example, the framework 120 can be formed by cutting, laser cutting, stamping, combinations thereof, etc. such that it is split to form the spines 122. In other examples, the spines 122 can be discrete members that converge at their fixed proximal ends 122-2.

The framework 120 can include a flexible, resilient material (e.g., a shape-memory alloy such as nickel-titanium, also known as Nitinol or stainless steel) that is shape set to be biased to bend outwardly to the expanded configuration, as shown in FIGs. 2 and 3. The spines 122 can be curved or extend approximately linearly in the outward direction to respective distal ends 122-1. Due to the flexible, resilient nature of the material, the spines 122 can also be moved to a collapsed configuration where the spines 122 extend approximately along/parallel to the longitudinal axis 60.

The end effector further includes a flexible membrane 130 connected to the framework 120 and extending to a distal end (see distal end 102-2, which is discussed in greater detail below). Specifically, the membrane 130 is connected to each spine 122 such that the membrane 130 and plurality of spines 122 are fixed relative to each other to define a single member. In some examples, each spine 122 has an associated membrane 130. In other examples, a singular membrane 130 is connected to two or more (e.g., all) of the spines 122. The spines 122 bias the membrane 130 to the expanded configuration and the membrane 130 is also movable therewith to the collapsed configuration. As seen in FIGs. 2-3, the distal end 130-1 of the membrane extends beyond the distal ends 122-1 of the spines 122. This membrane 130 serves to enhance the atraumaticity of the tip of the end effector 100 and to protect the subject from sharp edges. The membrane 130 can include a flexible biocompatible material, such as a polymer. It is noted that the flexible membrane 130 in FIGs. 4A-7 and 9 is depicted without cross-sectional hatching to more easily differentiate between components shown in the respective figures.

The membrane 130 can include one or more sheets/layers fused together proximate the framework 120 into a single, contiguous insulative mass 130. For example, the membrane 130 can include a first layer 132 that forms a distal-facing exterior surface of the end effector 100, a second layer 134 that forms a proximal-facing exterior surface of the end effector 100, and a third layer 136 disposed between the first layer 132 and the second layer 134. See, for reference, FIG. 4A, which is discussed in greater detail below.

In some examples, one or more of the layers of the membrane 130 (e.g., third layer 136) comprises a dielectric material. For examples, it can include high dielectric sheets/tiles, or it can be formed using high dielectric TPU doping. The various configurations shown in the figures provide differing levels of electrical insulation between electrodes on opposite sides of the membrane 130. The electrical insulation may be tailored to direct electric field lines, and thereby electroporation of cells within target tissue, between bipolar pairs of ablation electrodes on opposite sides of the membrane 130 during PFA application.

The membrane 130 can be heat formed around at least a portion of a first flexible circuit 110 (FIG. 4A, discussed in greater detail below), a second flexible circuit 150 (FIG. 4A, discussed in greater detail below), and the framework 120, making the end effector 100 easy to manufacture compared with other labor-intensive processes. The polymer can include TPU, silicone, or other heat formed or shaped material which lends itself to said heat forming.

In the expanded configuration, the membrane 130, by virtue of its connection to the spines 122, generally resembles a flower (e.g., a lilium flower). More specifically, the membrane 130 defines a plurality of leaves 102 (or petals), each leaf 102 being associated with a respective spine 122 such that, in the present example, there are eight leaves 102A-102H that correspond to each of the eight spines 122. Each leaf 102 has a proximal end 102-1 connected/coupled to the tubular member 80 and a free distal end 102-2 that, in the expanded configuration, extends curvilinearly outward from the longitudinal axis 60. Moreover, the leaves 102 can be equiangularly disposed about the longitudinal axis 60. The free distal ends 102-2 of the leaves are physically separated (i.e., unconnected) from adjacent free distal ends 102-2 of the leaves such that each leaf 102 is able to move independently relative to the other leaves 102.

Voids 114 space adjacent leaves 102 from one another. The voids 114 extend from the distal ends 102-2 of the leaves and terminate at a location intermediate the leaf distal ends 102-1 and the leaf proximal ends 102-1. The voids 114 can be sized, shaped, or otherwise configured to allow the end effector 100 to flex through a sheath catheter. The voids 114 can be sized, shaped, or otherwise configured to collapse or expand when the end effector 100 is pressed against a non-planar tissue surface to provide conformal contact to the non-planar tissue surface. It is recognized that consistent contact of ablation electrodes 161 to the non-planar tissue may produce improved lesions compared to less conformal electrode contact.

In some examples, the free distal ends 102-2 of the leaves 102 extend beyond the distal ends 122-1 of the spines 122 (i.e., the membrane 130 extends beyond the distal ends 122-1, as seen in FIGs. 2 and 3). In other examples, the free distal ends 102-2 of the leaves 102 are at approximately the same location as that of the free distal ends 122-1 of the spines (i.e., the termination of the membrane 130 is approximately flush the distal ends 122-2 of the spines 122).

Making reference to FIGs. 2-4A, each leaf 102 includes a first flexible circuit 110 and a second flexible circuit 150. As shown in FIG. 4A (which depicts one leaf 102A representative of all the leaves 102A-102H), each flexible circuit 110, 150 includes a flexible substrate 111, 151 (i.e., a first flexible substrate layer 111 and a second flexible substrate layer 151). The first flexible circuit 110 and second flexible circuit 150 of each leaf 102 each include a plurality of electrodes 160 electrically connected to the PIU 30 via electrical interconnections (e.g., traces). Each set of the flexible circuits 110, 150 are positioned near a respective spine 122, and each respective flexible substrate 111, 151 can include electrodes 160 thereon. While not explicitly illustrated, it is noted that the end effector 100 can also include other layers, such as a location sensing loop layer for sensing a position or shape of the end effector 100.

Each respective flexible substrate layer 111, 151 comprises a bio-compatible material. In some examples, each flexible substrate layer 111, 151 is formed entirely from or about entirely from the bio-compatible material. In some examples, the flexible substrate layer is formed from polyimide, copper, LCP, nitinol substrate, thermoplastic polyurethane (TPU), silicone, thermoset resin, or other polymeric substrates. In some examples, each flexible substrate layer 111, 151 described herein can be made primarily of polyimide. In other examples, it can be made of any of biocompatible polyimides, glass-reinforced epoxy laminate materials, copper, or graphene, alone or in combination.

The flexible circuits 110, 150 are disposed on the membrane 130 that extends around the longitudinal axis 60. More specifically, the first flexible circuit 110 is disposed on a first side (i.e., an upper side relative to the orientation of FIG. 4A) the membrane 130 and the second flexible circuit 151 is disposed on a second side (i.e., a lower side relative to the orientation of FIG. 4A) of the membrane 130 that is opposite the first side along a vertical axis 62 that is generally orthogonal to a distal second of the leaves 102.

Moreover, the membrane 130 can be contiguous to the contact surfaces of the electrodes 160 so that only the contact surfaces of at least a portion of the plurality of electrodes 160 are exposed to the ambient environment. The contact surfaces of the electrodes 160 can be flush with an outer surface of the membrane 130, recessed relative to the outer surface of the membrane 130, or protrude therefrom. As used herein, "ambient environment" refers to the external environment such as the organ in which the end effector 100 is deployed or in the operating theater prior to being deployed in the biological organ. The membrane 130 at least partially encapsulates or spaces the different layers of the end effector 100 (e.g., the flexible circuits 110, 150 (including the substrate layers 111, 151) and the framework 120) along the vertical axis 62. See, for example, FIG. 4A, which shows the flexible circuits 110, 150 spaced from the spine 122 via the third layer 136 of the membrane 130, which is disposed between the flexible circuits 110, 150 and the spines 122.

It is noted that not all of the electrodes 160 on the flexible circuits 110, 150 described herein need be exposed through the insulative material 130 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood.

With continued reference to FIGs. 2-4A, the electrodes 160 comprise plural ablation (or treatment) electrode sets 161 and diagnostic electrodes 165. Each ablation electrode set 161 is positioned proximal a respective spine 122 and on a respective set of first and second flexible circuits 110, 150.

Each flexible circuit 110, 150 and electrode of the respective ablation electrode set 161 is vertically spaced apart (along the vertical axis 62) from its associated/adjacent spine 122 by the membrane 130 (a portion of which is disposed between the spines 122 and the respective flexible circuits 110, 150). As can be seen in FIGs. 2-4A, each ablation electrode 161 is provided on a lateral side (i.e., in the left/right direction relative to the orientation of FIG. 4A) of its associated/adjacent spine 122 of its leaf 102 and extends along a predetermined length thereof.

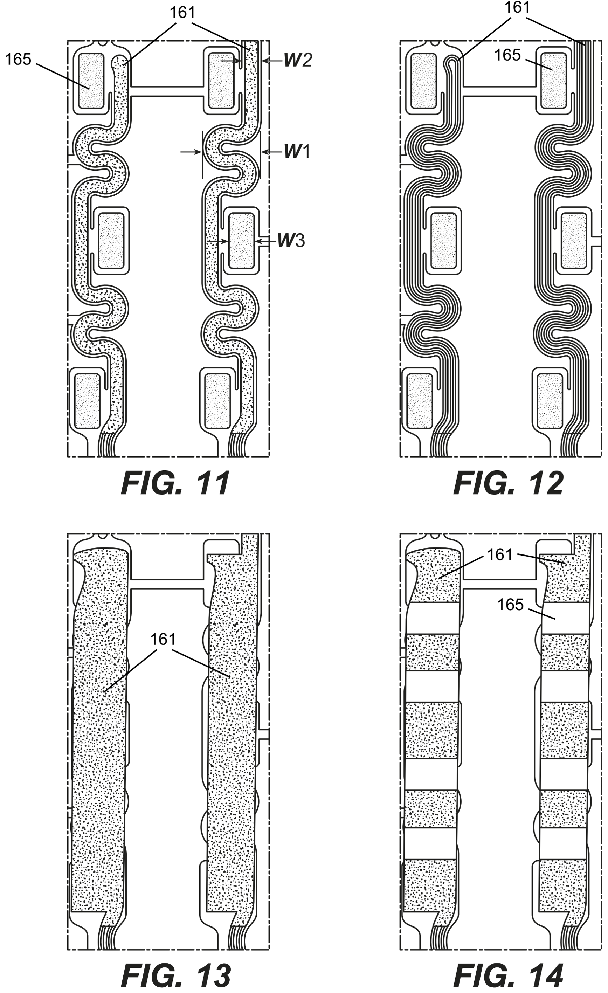

The left side of FIG. 4A is example electrode configuration on both sides (upper and lower) of the example end effector 100 including elongated ablation electrodes 161, each ablation electrode defining a treatment region. While not shown in this figure, the diagnostic electrodes 165 can be positioned in the two ablation electrode regions (see, e.g., FIG. 11 discussed below) or outside the treatment regions, tissue contact electrodes (discussed in greater detail below) positioned between the two upper ablation electrode regions, and a reference electrode (discussed in greater detail below) positioned in a region of the end effector 100 that does not contact tissue.

Each spine 122 can generally serve as a line of symmetry for its respective associated electrodes 160 (i.e., the electrodes 160 that are disposed proximal thereto). As illustrated, the electrode configuration is symmetric about the spine 122. Preferably, at least the ablation electrodes 161 are symmetric to each other with respect to the spine 122. In an alternative example, the electrode configuration may not be symmetric about the line of symmetry.

As seen in FIG. 4A, in some examples, each ablation electrode set 161 includes four ablation electrodes 161A-161D, including a first ablation electrode 161A, a second ablation electrode 161B, a third ablation electrode 161C, and a fourth ablation electrode 161D. A first pair of ablation electrodes 161A, 161B is disposed on the first flexible circuit 110 proximal to one another and their associated spine 122. Similarly, a second pair of ablation electrodes 161C, 161D is disposed on the second flexible circuit 150 proximal to one another and their associated spine 122, with the membrane 130 between them such that a mirror image configuration is defined. The first pair of ablation electrodes 161A, 161B is configured to be positioned against tissue during treatment. When the upper side of the end effector 100 is in contact with tissue, corresponding electrodes 161C, 161D on the opposite side are in contact with body fluid (e.g., blood) and can act as respective reference electrodes for those electrodes 161A, 161B in contact with tissue.

The illustrated portion of the end effector 100 has a left half which is left of the line of symmetry and a right half which is right of the line of symmetry. The first ablation electrode 161A defines a first electrode region, which is entirely on the left side of its associated spine 122. The second ablation electrode 161B defines a second electrode region, which is entirely in the right half of its associated spine 122. The third and fourth ablation electrodes 161C, 161D similarly define third and fourth electrode regions which are respectively entirely on the left and right sides of the spine 122.

Moreover, the membrane 130 extends laterally beyond and around the ablation electrodes 161A-161D and flexible circuit 110, 150 such that the ablation electrodes 161A-161D are offset from a lateral edge of their respective leaf 102. FIG. 4A depicts exemplary offset portions 112, 113 of the first flexible substrate 111 and FIG. 4B depicts exemplary offset portions 133 of the membrane that space the ablation electrodes 161 from edges of the leaves 102. This design, in conjunction with the voids 114 between the leaves 102, aids in preventing ablation electrodes 161 from adjacent leaves (e.g., as schematically depicted in FIG. 4B) from contacting and electrically shorting with one another.

The ablation electrodes 161A-161D can take a number of shapes/designs, such as a serpentine shape, provided as a strip, provided as a bar, or provided as an articulating bar. FIGs. 11-14 depict exemplary configurations thereof, which are described in greater detail below. In general, ablation electrodes 161C, 161D are illustrated on the second (lower) side opposite the ablation electrodes 161A, 161B on the first (upper) side of each leaf 102. The opposite ablation electrodes 161C, 161D overlap the ablation electrodes 161A, 161B on the first side preferably overlapping a majority of the ablation electrodes 161A, 161B on the first side 101a, and may be symmetric to the ablation electrodes 161C, 161D on the first side with respect to a plane of each leaf 102 when laid flat (e.g., as depicted in FIG. 4A for illustrative purposes). The cross-section of the end effector 100 is simplified to omit certain features, such as, but not limited to, electrical traces, for the sake of illustration. The ablation electrodes 161A-161D are illustrated sunken into the membrane 130, but may alternatively protrude therefrom on each respective side or be flush therewith (as discussed above). The ablation electrodes 161A-161D can have approximately equal surface area to each other.

In some examples, the ablation electrodes 161A-161D have a length that is at least half of a total radius of the end effector 100 as measured from the distal end 130-1 of the flexible membrane 130 to the longitudinal axis 60 when in the expanded configuration. In some examples, the ablation electrodes 161A-161D extend to approximately the distal end 102-1 of their respective leaves 102.

The ablation electrodes 161A-161D of each set are connected to the ablation energy generator 50. With the configuration detailed herein, a plurality of ablation configurations can be employed. In some examples, and as mentioned above the ablation electrodes 161C, 161D on the second side can function as respective reference electrodes for the respective opposite electrode 161A, 161B on the first side of the membrane 130 when the first side is in contact with tissue. Additionally, or alternatively, the ablation electrodes 161 on opposite sides can be paired to provide bipolar PFA electrical signals between the paired ablation electrodes 161. Additionally, or alternatively, electrodes 161 on the same side can be paired to provide bipolar PFA electrical signals between the paired electrodes. The bipolar PFA electrical signals may be monophasic or biphasic. It is noted that while each pair of first electrodes (161A, 161B) are described as discrete electrodes, it is within the scope of the claimed technology that both electrodes in each of the first pair (e.g., 161A, 161B) or the second pair (e.g., 161C, 161D) are electrically connected to form a single ablation electrode. Specifically, the first pair of electrodes 161A, 161B can be electrically connected together, via the spine 122 between the electrodes or at the generator 50, to define a single first electrode of the plurality of first electrodes disposed on the first surface of the membrane. Similarly, the second pair of electrodes 161C and 161D can be electrically connected together, via the spine 122 between them or at the generator 50, to define a single second electrode of the plurality of second electrodes disposed on the second surface of the membrane 130. It is further noted that each of the electrode 161A, 161B, 161C, 161D can be configured as a diagnostic electrode (which receives electrical signals from the tissues instead of transmitting electrical signals from the generator for ablation).

The illustrated end effector 100 includes optional diagnostic electrodes 165 on the first side of the membrane 130 that are electrically isolated from the ablation electrodes 161. The diagnostic electrodes 165 are configured to receive electrical signals from tissue to map cardiac tissue and detect arrhythmia. As illustrated, the diagnostic electrodes 165 are arranged as a pair with a first diagnostic electrode 165 on the left side of each spine 122 and a second diagnostic electrode 165 on a right side of each spine 122.

The end effector 100 can include optional tissue contact quality electrodes positioned in closely spaced pairs such that impedance measured across the respective tissue contact quality electrode pair indicates that electrodes of that pair are both in contact with tissue.

The end effector 100 includes an optional reference electrode (it is noted that, while primarily described as a diagnostic electrode, reference number 165 can also be considered to denote an exemplary reference electrode or any other electrode discussed herein) disposed on the first side of the membrane 130. Additionally, or alternatively, the end effector 100 can include a reference electrode similarly disposed on the second side membrane. The reference electrode(s) may be used for ECG gathering in either, unipolar, bipolar (split or close pair) or may be a reference. The number can be as few as four per side but as many as needed (and can fit with trace limitations). The signals from the reference electrode(s) may also be used for contact information to determine what portion of the paddle has contact or proximity to the tissue. The end effector 100 includes one or more sections which lack any ablation electrode. The reference electrode(s) may be disposed in this section. As discussed herein, an ablation electrode, diagnostic electrode, or tissue contact electrode may be used as a reference electrode for a corresponding electrode on the opposite side in contact with tissue. Each of these electrodes are positioned in the distal portion of the end effector 100 so that they may be positioned in contact with tissue if so desired by the physician 24. Preferably, the reference electrode is positioned such that the physician 24 is unable, or at least very unlikely, to position the reference electrode in contact with tissue but nevertheless in relatively close proximity to electrodes which are configured to contact tissue.

In some examples, and as mentioned above, at least the ablation electrodes 161, diagnostic electrodes 165, and tissue contact electrodes are flush with the outer surfaces of the membrane 130 to provide a first flush surface to the end effector 100 corresponding to the first side of the membrane 130 and a second flush surface to the end effector 100 corresponding to the second side of the membrane. The ablation electrodes 161 and any combination of other electrodes 165 (as well as the others discussed herein) may include an exposed conductive layer in a flexible printed circuit board, may include silver epoxy, or conductive ink.

Further details on exemplary configurations of the electrodes can be found with respect to the description of FIGs. 11-14.

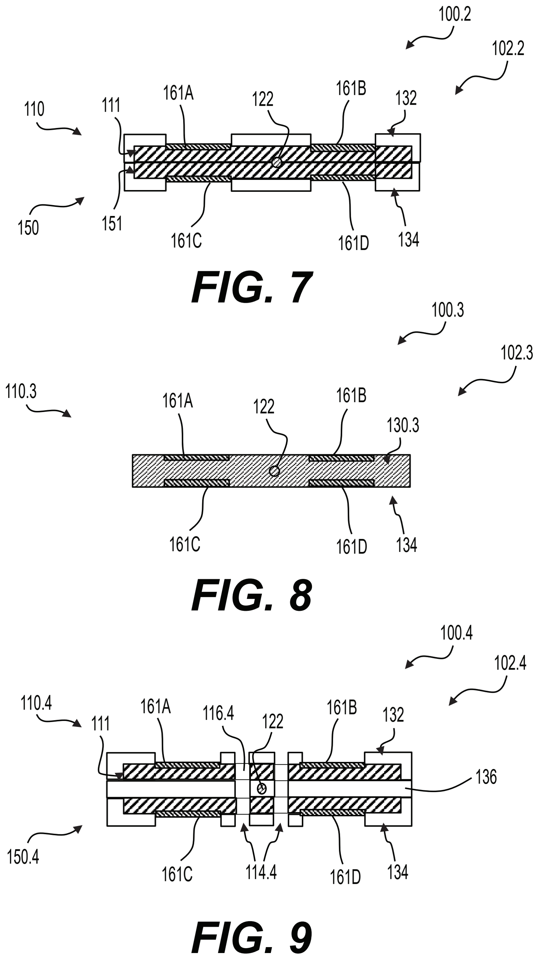

As discussed above, the membrane 130 can include a third layer 136 between the first/upper and second/lower sides of the end effector 100. The third layer 136 can include a polymeric body region extending a width of the respective region associated with each ablation electrode set 161 and spine 122. In some examples, the third layer 136 can be provided as respective thinned polymer fill-in regions that lack a framework or any electrical circuitry. Configured as such, the membrane 130 may provide additional electrical insulation between electrodes 160 on opposite sides of the end effector 100 (compared to examples that include openings, such as seen in FIG. 9 and discussed below) while maintaining sufficient flexibility for manipulation to position against tissue and transfer through a sheath. As mentioned above, this third layer 136 can also be embodied as a high dielectric layer between each side and extending around the end effector 100. The dielectric layer 136 is overlapping and parallel to each of the ablation electrodes 161A-161D. The high dielectric layer 136 can include a ceramic doped polymer to provide additional electrical insulation between electrodes 160 on opposite sides of the membrane 130 compared to polymer alone while still providing sufficient flexibility. Some portions of the end effector 100 can have the high dielectric layer 136, lacking a framework, and lacking electrical circuitry. In some examples, the third layer 136 can be embodied as high dielectric tiles between each side and configured to overlap to collapse with the end effector 100 into a delivery sheath. The high dielectric tiles can include ceramic plates that extend longitudinally through each segment of the body and overlap between segments. The high dielectric tiles or longitudinally extending ceramic plates may be angled with respect to a plane defined by the membrane 130 when laid flat such that the tiles/plates are configured to overlap, longitudinal side on longitudinal side upon retraction of the end effector into a sheath.

FIGs. 5A-5C depict exemplary ablation configurations for application of bipolar PFA electrical pulses in examples where each leaf 102 of the end effector 100 includes four ablation electrodes 161A-161D. In these figures, the first and second ablation electrodes 161A, 161B face outwardly towards tissue while the third and fourth ablation electrodes 161C, 161D (which are disposed in the volume V) face away from the tissue. In use, the first and second ablation electrodes 161A, 161B can contact the tissue (or be disposed immediately adjacent thereto), and the ablation energy generator 50 selectively activates pairs of the ablation electrodes to apply pulse field ablation pulses. In some examples, the pulses have a voltage of approximately 600 volts (V) to about 1,200 V. In other examples, the pulses have a voltage from 600 V to 2,600 V. In some examples, a bipolar PFA electrical signal includes 60 pulses with a magnitude of approximately 1,200 V and a total duration of approximately 4 seconds. In some examples, the bipolar pulses may include an interpulse delay of approximately 2 microseconds. The pattern of ablation electrode pairings can be adapted based on the total number of ablation electrodes in the end effector 100 as discussed in greater detail as understood by a person skilled in the pertinent art informed by the disclosure herein.

FIG. 5A depicts one such exemplary ablation configuration. In this example, the ablation electrodes 161A-161D are activated diagonally/cross-body and sequentially. The generator 50 can alternatingly energize a first pair of the ablation electrodes 161 to apply pulses between the first ablation electrode 161A(+) and fourth ablation electrode 161D(-), followed by energizing a second pair of the ablation electrodes 161 to apply a pulse between the second ablation electrode 161B(+) and the third ablation electrode 161C(-), such that only one pair of ablation electrodes 161 of each set is activated at a time.

In this example, the ablation electrodes 161 in a pair are symmetric with respect to the spine 122 of their respective leaf 102 and are on opposite sides of the membrane 130. A voltage is applied across electrodes 161 in pairs of electrode regions in which, for each pair, ablation electrodes in a first electrode region is in contact with tissue and the other electrode region is on an opposite side of the end effector 100 and across the spine 122 from the first electrode region. FIG. 5A illustrates ablation electrode 161A having a positive charge while the paired ablation electrode 161D has a negative charge. This illustrates a positive voltage pulse configured to induce electroporation in tissue in contact with the upper side of the membrane 130. A biphasic pulse may be applied in which the polarity switches or alternates between as shown in FIG. 5A to one in which positive charge is on the ablation electrode 161D not in contact with tissue while negative charge is on the ablation electrode 161A in contact with tissue. A train of bipolar pulses (which may include biphasic or monophasic pulses) can be applied between the bipolar ablation electrode pair 161A, 161D as shown in FIG. 5A, then subsequently, a train of bipolar pulses can be applied between the other bipolar ablation electrode pair 161B, 161C in the same manner as described above.

Put another way, FIG. 5A depicts an end effector 100 that is configured to provide electrical pulses 600-1,200 V in amplitude between bipolar pairs which include an ablation electrode (e.g., first ablation electrode 161A) on the upper side of the membrane 130 and in contact with tissue, and an ablation electrode (e.g., fourth ablation electrode 161D) on the lower side of the membrane 130 and not in contact with tissue. Similarly, ablation electrodes 161B, 161C can be paired.

In some examples, cardiac electrical signals may be measured from one or more diagnostic electrode(s) disposed on the side of the membrane 130 in contact with tissue. In some embodiments, tissue contact may be measured from a pair of tissue contact electrodes disposed on the side of the membrane 130 in contact with tissue. In some examples, a reference electrical signal may be measured from a reference electrode disposed on the side of the membrane 130 in contact with tissue, wherein the reference electrode itself is not in contact with tissue.

FIG. 5B depicts another exemplary ablation configuration. In this example, rather than applying the pulses diagonally, the pulses are applied vertically (relative to the orientation of FIG. 5B) such that the ablation electrodes in each pair are overlapping and opposite sides of the membrane 130. The generator 50 can simultaneously or alternatingly energize a first pair of the ablation electrodes 161 to apply pulses between the first ablation electrode 161A(+) and third ablation electrode 161C(-), as well as energize a second pair of the ablation electrodes 161 to apply a pulse between the second ablation electrode 161B(+) and the fourth ablation electrode 161D(-). A voltage is applied across ablation electrodes 161A, 161B in contact with tissue to ablation electrodes 161C, 161D on an opposite side of the membrane 130 and not in contact with tissue. A biphasic pulse may be applied in which the polarity switches or alternates between as shown in FIG. 5B to one in which positive charge is on ablation electrodes 161C, 161D not in contact with tissue while negative charge is on ablation electrodes 161A, 161B in contact with tissue. A train of bipolar pulses can be applied between the bipolar ablation electrode pairs. As illustrated, both ablation electrodes 161A, 161B in contact with tissue can be activated simultaneously. Alternatively, a train of bipolar pulses may be applied to the first pair (161A, 161C) of ablation electrodes while the second pair (161B, 161D) is deenergized, and subsequently a train of bipolar pulses may be applied to the second pair of ablation electrodes while the first pair is deenergized.

FIG. 5C depicts yet another exemplary ablation configuration. In this example, rather than applying the pulses diagonally or vertically, the pulses are applied horizontally (relative to the orientation of FIG. 5B) across the upper pair of electrodes 161 on each leaf 102. In other words, the ablation electrodes 161 in each pair are symmetric with respect to the spine 122 and on the same side of the membrane 130. Specifically, the generator 50 can energize the upper pair of the ablation electrodes 161 to apply pulses between the first ablation electrode 161A(+) and second ablation electrode 161B(-), while the third and fourth ablation electrodes 161C, 161D are not activated. As illustrated, a voltage is applied across ablation electrodes 161A, 161B on the side membrane 130 of each leaf 102 in contact with tissue. The ablation electrodes 161C, 161D not in contact with tissue can function as reference electrodes. A biphasic pulse may be applied in which the polarity switches or alternates between as shown in FIG. 5C to one in which positive charge is on the right ablation electrode 161B while negative charge is on the left ablation electrode 161A. A train of bipolar pulses can be applied between the bipolar ablation electrode pair 161A, 161B.

It is noted that cross-leaf ablative pulses with similar patterns to those described with respect to FIGs. 5A and 5C can be employed without departing from the spirit and scope of the present disclosure. By way of example, and with reference to FIG. 2, a train of bipolar pulses (which, as mentioned above, may include biphasic or monophasic pulses) can be applied between an ablation electrode 161 on the first leaf 102A and an ablation electrode 161 on the second leaf 102B, followed by a train of bipolar pulses between an ablation electrode 161 on the third leaf 102C and an ablation electrode 161 on the fourth leaf 102D, followed by a train of bipolar pulses between an ablation electrode 161 on the fifth leaf 102E and an ablation electrode 161 on the sixth leaf 102F, followed by a train of bipolar pulses between an ablation electrode 161 on the seventh leaf 102G and an ablation electrode 161 on the eighth leaf 102H. Of course, other cross-leaf ablative patterns can be used. For another example, and with continued reference to FIG. 2, a train of bipolar pulses (which may include biphasic or monophasic pulses) can be applied between an ablation electrode 161 on the first leaf 102A and an ablation electrode 161 on the fifth leaf 102E, followed by a train of bipolar pulses between an ablation electrode 161 on the second leaf 102B and an ablation electrode 161 on the sixth leaf 102F, followed by a train of bipolar pulses between an ablation electrode 161 on the third leaf 102C and an ablation electrode 161 on the seventh leaf 102G, followed by a train of bipolar pulses between an ablation electrode 161 on the fourth leaf 102D and an ablation electrode 161 on the eighth leaf 102H.

Of course, other cross-region ablative patterns can be used. For another example, and with continued reference to FIG. 2, a train of bipolar pulses (which may include biphasic or monophasic pulses) can be applied between one or more ablation electrodes 161 on the first leaf 102A and one or more ablation electrodes 161 in the fifth leaf 102E, followed by a train of bipolar pulses between one or more ablation electrodes 161 in the second leaf 102B and one or more ablation electrodes 161 in the sixth leaf 102F, followed by a train of bipolar pulses between one or more ablation electrodes 161 in the third leaf 102C and one or more ablation electrodes 161 in the seventh leaf 102G, followed by a train of bipolar pulses between one or more ablation electrodes 161 in the fourth leaf 102D and one or more ablation electrodes 161 in the eighth leaf 102H. Similar techniques can be employed for the example of FIG. 2A.

In even further examples, cross-leaf ablative pulses can have even further bipole patterns that employ two or more leaves as the positive electrode in the bipole. By way of example, and with reference to the example of FIG. 2, a train of bipolar pulses can be applied between ablation electrodes 161 in the first and second leaves 102A, 102B (functioning as the positive electrode in the bipole) and ablation electrodes 161 in the fifth and sixth leaves 102E, 102F (functioning as the negative electrode in the bipole), followed by a train of bipolar pulses applied between ablation electrodes 161 in the second and third leaves 102B, 102C (functioning as the positive electrode in the bipole) and ablation electrodes 161 in the sixth and seventh leaves 102F, 102G (functioning as the negative electrode in the bipole), followed by a train of bipolar pulses applied between ablation electrodes 161 in the third and fourth leaves 102C, 102D (functioning as the positive electrode in the bipole) and ablation electrodes 161 in the seventh and eighth leaves 102G, 102H (functioning as the negative electrode in the bipole), followed by a train of bipolar pulses applied between ablation electrodes 161 in the fourth and fifth leaves 102D, 102E (functioning as the positive electrode in the bipole) and ablation electrodes 161 in the eighth and first leaves 102G, 102A (functioning as the negative electrode in the bipole).

The above ablation configuration examples are non-limiting and are merely intended to elucidate certain ways in which the presently described technology can be implemented. In all of the above-described examples, the goal of the configuration of the ablation bipoles and sequencing is to achieve a circumferential lesion without needing to reposition the end effector 100.

Having described various exemplary ablation patterns that can be employed, the present disclosure now turns to exemplary alternative physical forms the electrodes 160 or the overall end effector 100 can take. Unless explicitly noted to the contrary, it will be appreciated that the previously described ablation configurations can be applied to any of the following exemplary end effectors as well.

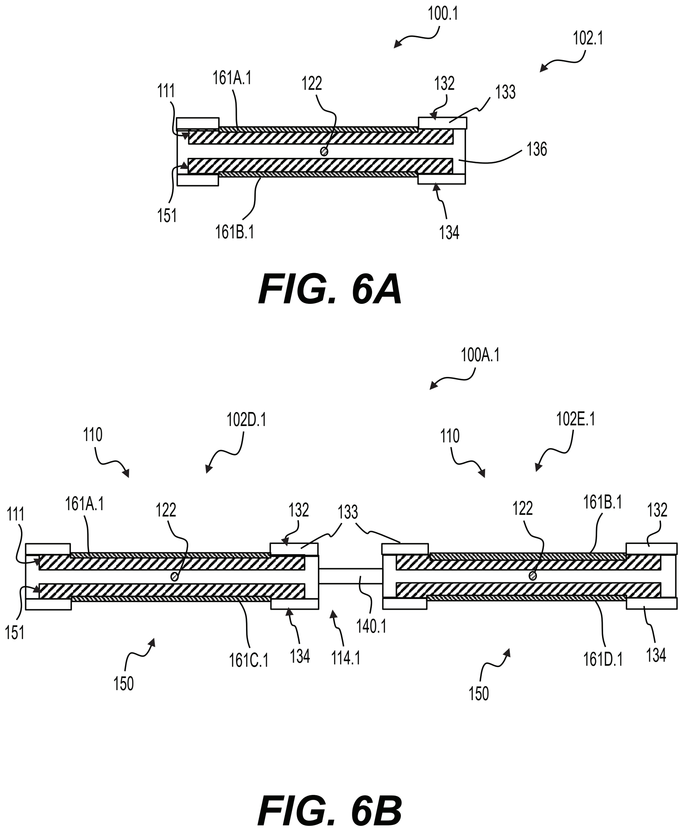

FIG. 6A depicts a variant 100.1 of the above-described end effector 100. This example is identical to the previously described one with the exception that, rather than providing a set of four ablation electrodes 161 proximal each spine 122 on each leaf 102, a single pair of ablation electrodes is provided. Specifically, a first ablation electrode 161A.1 is disposed on the first flexible substrate 111 and second ablation electrode 161B.1 is disposed on the second flexible substrate 151 in a mirror configuration. This example can employ the ablation configuration discussed with respect to FIG. 5B or the exemplary cross-leaf ablative patterns described in the preceding paragraphs. It is additionally noted that, in some examples, a single ablation electrode 161A.1 can be provided on the upper side of each leaf 102.1, with the above-described cross-leaf ablative pulses being employed to achieve IRE.

FIG. 6B depicts a variant 100A.1 of the end effector 100.1 of FIG. 6A. This example is identical to the variant 100.1 discussed above, but provides a connecting bridge 140.1 along an intermediate portion of adjacent leaves (e.g., leaves 102D.1, 102E.1 shown in FIG. 6B including ablation electrodes 161A.1, 161C.1 and ablation electrodes 161B.1, 161D.1 respectively). In some examples, the bridge 140.1 can be provided as respective thinned polymer fill-in regions that lack a framework or any electrical circuitry. The bridge 140.1 can provide additional predictability to the shape of the end effector 100A.1 in the expanded configuration (and thus, more predictable distances/relative positioning between the electrodes 160 of the end effector 100).

FIG. 7 depicts yet another variant 100.2 of the above-described end effector 100. This variant 100.2 can include leaves 102.2 that are configured identically to that of the end effector 100, with the third layer 136 of the membrane omitted such that the two flexible substrates 111, 151 can be provided directly on the spines 122 or in contact with one another (rather them otherwise being spaced apart via an intermediate membrane layer).

FIG. 8 depicts yet another variant 100.3 of the above-described end effector 100. In this example, the membrane 130.3 of each leaf 102.3 functions as the flexible circuit 110.3, with the first and second ablation electrodes 161A, 161B and second and third ablation electrodes 161C, 161D being provided on opposing surfaces thereof.

Reference is now made to FIG. 9, which depict yet another variant 100.4 of the above-described end effector 100. In this example, each leaf 102.4 is configured with voids 114.4 defined therethrough (i.e., through the membrane 130.4 and flexible circuits 110.4, 150.4), with each void 114.4 having a closed perimeter bounded by the membrane 130.4. Moreover, the voids 114.4 are defined along the vertical axis 62 and between (relative to a lateral direction) the first/third ablation electrodes 161A/161C and the second/fourth ablation electrodes 161B/161D of the leaf 102.4. The distal ends of the membranes 130.4 have connecting material 116.4 that partially defines the voids 114.4 and maintains connection between the sections of the leaves 102.4 separated by the voids 114.4. These voids 114.4 can be employed, for example, to provide for a gap in dielectric material (e.g., layer 136) on the leaves 102.4, while the connecting material 116.4 aids in maintaining the relative spacing between the ablation electrodes 161 on the respective leaves 102.

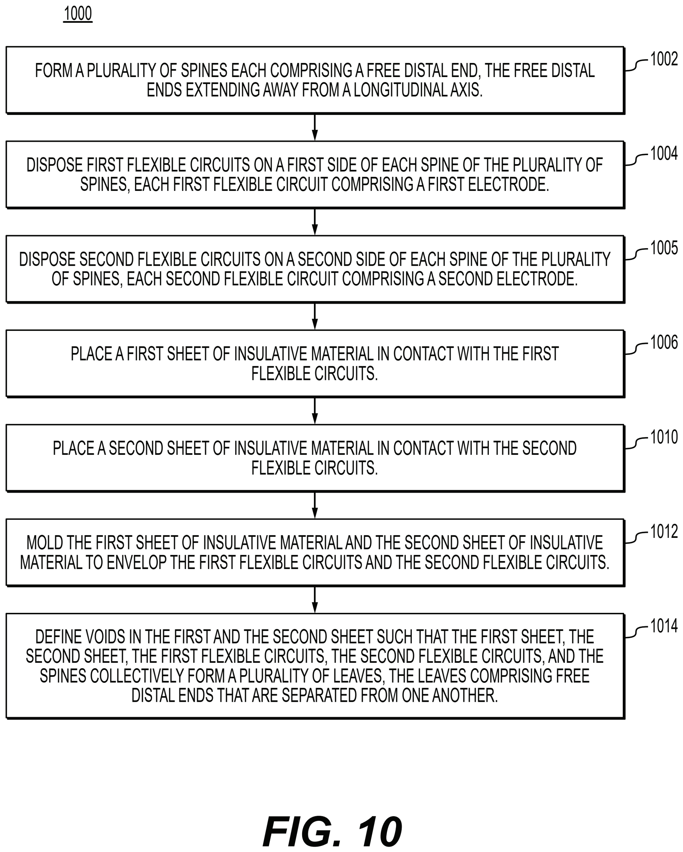

FIG. 10 is a flow chart depicting a method 1000 of making an end effector 100 in accordance with the present disclosure. A plurality of spines are formed 1002, each comprising a free distal end, the free distal ends extending away from a longitudinal axis. First flexible circuits are disposed 1004 on a first side of each spine of the plurality of spines, each first flexible circuit comprising a first flexible substrate and a first electrode. Second flexible circuits are disposed 1006 on a second side of each spine of the plurality of spines, each second flexible circuit comprising a second flexible substrate and a second electrode. A first sheet of insulative material is placed 1008 in contact with the first flexible circuits. A second sheet of insulative material is placed 1010 in contact with the second flexible circuits. The first sheet and the second sheet are molded 1012 to envelop the first flexible circuits and the second flexible circuits. Voids are defined 1014 in the first and the second sheet such that the first sheet, the second sheet, the first flexible circuits, the second flexible circuits, and the spines collectively form a plurality of leaves, with the leaves comprising free distal ends that are separated from one another.

In some examples, the method includes, prior to molding the first sheet of insulative material and the second sheet of insulative material, placing a third sheet of insulative material between the first flexible circuits and the second flexible circuit and in contact with the spine, and molding the third sheet of insulative material such that each first flexible circuit and each second flexible circuit is spaced apart from the respective spines.

Turning now to FIGs. 11-14, alternative/exemplary electrode configurations are depicted therein. In some examples, the electrodes can have a fishbone configuration. Examples of electrodes with fishbone configurations are disclosed in U.S. Patent No. 11,642,165B2, which is incorporated herein by reference in its entirety. Other exemplary designs are detailed below. In general, each ablation electrode can be designed with shape based on trade-off between total edge area, total contact area, and open space for non-ablation electrodes. It is recognized that a larger ratio of surface area to perimeter may effectively deliver the ablation energy while reducing the chance of electrical arcing. It is also recognized that it is desirable to avoid arcing. Conversely, serpentine electrodes increase mechanical flexibility of the paddle. It is recognized that flexibility of the electrode may result in improved tissue contact and catheter longevity, but the geometry of the serpentine electrode has a lower area to perimeter ratio compared to a solid electrode.

FIG. 11 is an illustration of a first electrode configuration for the example end effector 100 including one example configuration of a treatment/ablation electrode 161 and diagnostic electrodes 165. In the illustrated example, each ablation electrode 161 is divided into two (or pairs of) longitudinally elongated segments that extend along their associated spine 122/leaf 102. In this example, longitudinally elongated segments on a left side of the spine 122 form a first ablation electrode 161, and longitudinally elongated segments on a right side of the spine 122 form a second ablation electrode 161. By way of example, with this configuration, the leaf 102 of the example of FIG. 4A would have eight elongated segments in total (per each associated spine 122/leaf 102), with four on the upper side and four on the lower side of the membrane 130. It will be appreciated, of course, that in some examples, the aforementioned four electrodes 161A-161D each have a single elongated segment such that there can be four elongated segments in total (per each leaf 102). In some examples, the total area of each ablation electrode 161 or ablation electrode segment within a respective electrode region (discussed above with respect to FIG. 4A) is approximately 7.5 mm². In some examples, such as a configuration like that of FIG. 4A that implements the pairs of segments of FIG. 11, one pair of these electrode segments on a respective leaf can be connected together by the generator 50 with the same charge (e.g., a positive charge), while another pair of these electrode segments can be connected together by the generator 50 and having the same charge (e.g., a negative charge), such that a biphasic pulse field is provided between these two pairs of ablation electrode segments. In alternative examples, these adjacent elongated segments of each ablation electrode 161 (when in contact with tissue) can be disconnected from one other and separately connected to the generator 50 so that a biphasic pulse field can be provided between these two tissue contacting ablation electrode segments in a bipolar configuration to allow the flow of electrons between the ablation electrode segments.

In general, the ablation electrode 161 can include one or more serpentine longitudinally extending segments (in the illustrated example of FIG. 11, there are two, as mentioned above) with a total width W1 and a path width W2. The example in FIG. 11 includes six diagnostic electrodes 165 per ablation electrode 161. The end effector 100 may include one, two, three, four, five, or six diagnostic electrodes 165 per ablation electrode 111. Each diagnostic electrode is similarly sized with a width W3 that is approximately one and a half times the path width W2 of the ablation electrode 161 and approximately one half the total width W1 of an elongated segment of the ablation electrode 161. The diagnostic electrodes 165 are surrounded on two or three sides by a respective elongated segment of the ablation electrode 161.

FIG. 12 is an illustration of a second electrode configuration for the example end effector 100 including ablation electrodes 161 having elongated segments which are respectively segmented into small parallel stripes and diagnostic electrodes 165 positioned alongside the elongated ablation electrode segments. Each of the elongated segments has multiple electrically conductive stripes running parallel to each other to form an overall shape of a respective elongated segment that is similar to as shown in FIG. 11. Other ablation electrode shapes illustrated herein, and alternatives thereto as understood by a person skilled in the pertinent art may also be divided into multiple electrically conductive stripes running parallel to each other similar to as shown in FIG. 12. This configuration increases the total edge length of the ablation electrode to provide a different electric field profile to tissue during PFA than a similarly shaped ablation electrode that is solid (e.g., as shown in FIG. 11).

FIG. 13 is an illustration of a third example electrode configuration for the example end effector 100 including rectangular elongated segments of the ablation electrode 161 without diagnostic electrodes 165. The entire width W1 of the electrode segment is utilized for the ablation electrode 161 to maximize surface area of the ablation electrode 161. Diagnostic electrodes can be disposed elsewhere on the flexible membrane 130.

FIG. 14 is an illustration of a fourth example electrode configuration for the example end effector 100 including rectangular elongated segments of the ablation electrode 161 interrupted by diagnostic electrodes 165. Compared to the continuous rectangular electrode segments shown in FIG. 13, the segmented elongated ablation electrode segments can have greater flexibility for the membrane 130 to flex, at the expense of total ablation electrode area.

In accordance with all of the above-described examples, the end effector 100 preferably includes a total of two to eight ablation electrodes 161 per associated spine 122. The end effector 100 preferably includes exactly two, three, or four ablation electrodes (or electrode segment) per side leaf 102. Each ablation electrode 161 preferably overlaps a corresponding ablation electrode 161 on the opposite side of the membrane 130 so that the ablation electrodes are symmetric with respect to a plane defined by the membrane 130 when laid flat (e.g., pre-assembly to its umbrella shaped configuration). Fewer ablation electrodes can be accomplished by electrically connecting combinations of ablation electrodes within the end effector 100 or elsewhere within the catheter 14. Increasing the number of ablation electrodes 161 can be accomplished by splitting apart an ablation electrode 161 into two portions that are electrically insulated from each other in the catheter 14 and configured to be independently activated by the generator 50. For example, the cross-sectional line 4B-4B in FIG. 2 could serve as a dividing line in an example in which the ablation electrode 161A is segmented into two electrodes: a distal ablation electrode (portion of 161A to the left of line 4B-4B in FIG. 2) and a proximal ablation electrode (portion of 161A to the right of line 4B-4B in FIG. 2). Likewise, the ablation electrodes 161B, 161C, 161D can be divided in a similar manner.

In summary, ablation electrodes 161 can be paired in various pairing combinations to provide bipolar PFA electrical signals between treatment electrodes in a pair. Pairs can be activated simultaneously or sequentially in various combinations to achieve PFA of target tissue as understood by a person skilled in the pertinent art informed by the disclosure herein. The example treatment electrode configurations illustrated and described herein are non-limiting and numerous other treatment electrode configurations are possible. In each treatment electrode configuration, treatment electrodes can be paired following the same concepts as outlined in the foregoing disclosure.

The disclosed technology described herein can be further understood according to the following clauses:
Clause 1. An end effector of a medical probe, the medical probe comprising a tubular member extending along a longitudinal axis and including a distal portion and a proximal portion, the end effector comprising: a plurality of spines coupled to the distal portion with each spine including a proximal spine end coupled to the distal portion of the tubular member and a free distal end; and a membrane connected to the respective plurality of spines so that the membrane and the plurality of spines are fixed relative to each other to define a single member, the membrane defining a plurality of leaves, each leaf including a proximal end configured to be connected to a distal end of the tubular member and a free distal end that, in an expanded configuration, extends curvilinearly outward from the longitudinal axis and is separated from adjacent free distal ends of adjacent leaves of the plurality of leaves, each leaf comprising: a first flexible circuit disposed on the membrane on a first side of the spine, the first flexible circuit comprising a first electrode; and a second flexible circuit disposed on the membrane on a second side of the respective spine, the second flexible circuit comprising a second electrode.
Clause 2. The end effector of clause 1, the plurality of leaves being equiangularly disposed about the longitudinal axis.
Clause 3. The end effector of any one of clauses 1-2, each leaf being biased to the expanded configuration by the respective spine.
Clause 4. The end effector of any one of clauses 1-3, the end effector being moveable between a collapsed configuration, in which each leaf is disposed generally along the longitudinal axis, and the expanded configuration.
Clause 5. The end effector any one of clauses 1-4, the first flexible circuit and the second flexible circuit of each leaf being spaced apart from the respective spine along a vertical axis.
Clause 6. The end effector of clause 5, a portion of the membrane of each leaf being disposed between the respective spine and the respective first flexible circuit and between the respective spine and the respective second flexible circuit.
Clause 7. The end effector of any one of clauses 5-6, each leaf comprising at least one void defined therethrough along the vertical axis and between the first electrode and the second electrode in a lateral direction of the respective leaf.
Clause 8. The end effector of any one of clauses 1-7, the membrane comprising a flexible biocompatible polymer material.
Clause 9. The end effector of any one of clauses 1-8, the membrane comprising a plurality of layers, with at least one of the layers comprising an insulative material.
Clause 10. The end effector of clause 9, the plurality of layers of each leaf comprising at least one layer comprising a dielectric material.
Clause 11. The end effector of any one of clauses 1-10, the first electrode and the second electrode of each leaf being flush with an outer surface of the membrane.
Clause 12. The end effector of any one of clauses 1-11, the first electrode and the second electrode of each leaf being offset from a lateral edge of the respective leaf.
Clause 13. The end effector of any one of clauses 1-12, the first flexible circuit of each leaf comprising a third electrode and the second flexible circuit of each leaf comprising a fourth electrode.
Clause 14. The end effector of any one of clauses 1-13, the plurality of leaves comprising pairs of leaves, each pair being connected by a bridge at an intermediate portion of the respective leaves of the pairs of leaves.
Clause 15. The end effector of any one of clauses 1-14, the plurality of leaves comprising five leaves.
Clause 16. The end effector of any one of clauses 1-15, the plurality of leaves comprising eight leaves.
Clause 17. The end effector of any one of clauses 1-16, the first electrode and the second electrode being ablation electrodes, and each leaf comprising one or more diagnostic electrodes.
Clause 18. The end effector of any one of clauses 1-17, the first electrode and the second electrode each comprising an elongated segment comprising a plurality of conductive stripes running parallel to each other to form an overall shape of the elongated segment.
Clause 19. A medical system comprising: a medical probe comprising an elongated tubular member and an end effector connected to a distal end of the elongated probe body, the tubular member and the end effector extending along a longitudinal axis, the end effector comprising: a plurality of leaves, each leaf including a proximal end connected to a distal end of the tubular member and a free distal end that, in an expanded configuration, extends curvilinearly outward from a longitudinal axis of the end effector and is separated from adjacent free distal ends of adjacent leaves of the plurality of leaves, each leaf comprising: a spine with a free distal end; a membrane connected to the respective spine so that the spine is fixed relative to the membrane; a first flexible circuit disposed on the membrane on a first side of the spine; a second flexible circuit disposed on the membrane on a second side of the respective spine, the second flexible circuit comprising a second electrode; a first ablation electrode disposed on the first flexible circuit; and a second ablation electrode disposed on either the first flexible circuit or the second flexible circuit; and an ablation generator configured to provide ablation pulses between the first ablation electrode and the second ablation electrode of each leaf.
Clause 20. The medical system of clause 19, the second ablation electrode of each leaf being disposed on the second flexible circuit of the respective leaf.
Clause 21. The medical system of clause 20, each leaf further comprising a third ablation electrode disposed on the first flexible circuit and a fourth ablation electrode disposed on the second flexible circuit, the ablation generator being configured to provide ablation pulses between the third ablation electrode and the fourth ablation electrode of each leaf.
Clause 22. The medical system of clause 21, the first ablation electrode and the second ablation electrode of each leaf being disposed on opposing lateral sides, relative to the respective spine, of the respective leaf, and the third ablation electrode and the fourth ablation electrode of each leaf being disposed on opposing lateral sides, relative to the respective spine, of the respective leaf.
Clause 23. The medical system of clause 21, the first ablation electrode and the second ablation electrode of each leaf being disposed on a first lateral side, relative to the respective spine, of the respective leaf, and the third ablation electrode and the fourth ablation electrode of each leaf being disposed on a second lateral side, relative to the respective spine, of the respective leaf.
Clause 24. The medical system of clause 19, the second ablation electrode of each leaf being disposed on the first flexible circuit of the respective leaf.
Clause 25. The medical system of clause 24, each leaf further comprising a third ablation electrode disposed on the second flexible circuit and a fourth ablation electrode disposed on the second flexible circuit, the ablation generator being configured to provide ablation pulses between the third ablation electrode and the fourth ablation electrode of each leaf.
Clause 26. The medical system of clause 25, the first ablation electrode and the second ablation electrode of each leaf being disposed on opposing lateral sides, relative to the respective spine, of the respective leaf, and the third ablation electrode and the fourth ablation electrode of each leaf being disposed on opposing lateral sides, relative to the respective spine, of the respective leaf.
Clause 27. The medical system of clause 19, the ablation generator being configured to provide ablation pulses between different leaves of the plurality of leaves.
Clause 28. A method of manufacturing an end effector for a medical probe, the method comprising: forming a plurality of spines each comprising a free distal end, the free distal ends extending away from a longitudinal axis; disposing first flexible circuits on a first side of each spine of the plurality of spines, each first flexible circuit comprising a first flexible substrate and a first electrode; disposing second flexible circuits on a second side of each spine of the plurality of spines, each second flexible circuit comprising a second flexible substrate and a second electrode; placing a first sheet of insulative material in contact with the first flexible circuits; placing a second sheet of insulative material in contact with the second flexible circuits; molding the first sheet and the second sheet to envelop the first flexible circuits and the second flexible circuits; and defining voids in the first and the second sheet such that the first sheet, the second sheet, the first flexible circuits, the second flexible circuits, and the spines collectively form a plurality of leaves, the leaves comprising free distal ends that are separated from one another.
Clause 29. The method of clause 28, further comprising: prior to molding the first sheet of insulative material and the second sheet of insulative material, placing a third sheet of insulative material between the first flexible circuits and the second flexible circuit and in contact with the spine; and molding the third sheet of insulative material such that each first flexible circuit and each second flexible circuit is spaced apart from the respective spines.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

To the extent that any materials incorporated by reference herein contain similar terms but differ in definition or description, it will be appreciated that the definitions or descriptions provided herein are to be used in understanding the technology disclosed herein.

## Claims

1. An end effector of a medical probe, the medical probe comprising a tubular member extending along a longitudinal axis and including a distal portion and a proximal portion, the end effector comprising:
a plurality of spines coupled to the distal portion with each spine including a proximal spine end coupled to the distal portion of the tubular member and a free distal end; and
a membrane connected to the respective plurality of spines so that the membrane and the plurality of spines are fixed relative to each other to define a single member, the membrane defining a plurality of leaves, each leaf including a proximal end configured to be connected to a distal end of the tubular member and a free distal end that, in an expanded configuration, extends curvilinearly outward from the longitudinal axis and is separated from adjacent free distal ends of adjacent leaves of the plurality of leaves, each leaf comprising:
a first flexible circuit disposed on the membrane on a first side of the spine, the first flexible circuit comprising a first electrode; and
a second flexible circuit disposed on the membrane on a second side of the respective spine, the second flexible circuit comprising a second electrode.

2. The end effector of claim 1, the plurality of leaves being equiangularly disposed about the longitudinal axis.

3. The end effector of claim 1 or claim 2, each leaf being biased to the expanded configuration by the respective spine.

4. The end effector of any preceding claim, the end effector being moveable between a collapsed configuration, in which each leaf is disposed generally along the longitudinal axis, and the expanded configuration.

5. The end effector of any preceding claim 1, the first flexible circuit and the second flexible circuit of each leaf being spaced apart from the respective spine along a vertical axis.

6. The end effector of claim 5, a portion of the membrane of each leaf being disposed between the respective spine and the respective first flexible circuit and between the respective spine and the respective second flexible circuit and/or each leaf comprising at least one void defined therethrough along the vertical axis and between the first electrode and the second electrode in a lateral direction of the respective leaf.

7. The end effector of any preceding claim, the membrane comprising a plurality of layers, with at least one of the layers comprising an insulative material, optionally the plurality of layers of each leaf comprising at least one layer comprising a dielectric material.

8. The end effector of any preceding claim, the first electrode and the second electrode of each leaf being flush with an outer surface of the membrane.

9. The end effector of any preceding claim, the first electrode and the second electrode of each leaf being offset from a lateral edge of the respective leaf.

10. The end effector of any preceding claim, the first flexible circuit of each leaf comprising a third electrode and the second flexible circuit of each leaf comprising a fourth electrode.

11. The end effector of any preceding claim, the plurality of leaves comprising pairs of leaves, each pair being connected by a bridge at an intermediate portion of the respective leaves of the pairs of leaves.

12. The end effector of any preceding claim, the first electrode and the second electrode each comprising an elongated segment comprising a plurality of conductive stripes running parallel to each other to form an overall shape of the elongated segment.

13. A medical system comprising:
a medical probe comprising an elongated tubular member and an end effector connected to a distal end of the elongated probe body, the tubular member and the end effector extending along a longitudinal axis, the end effector comprising:
a plurality of leaves, each leaf including a proximal end connected to a distal end of the tubular member and a free distal end that, in an expanded configuration, extends curvilinearly outward from a longitudinal axis of the end effector and is separated from adjacent free distal ends of adjacent leaves of the plurality of leaves, each leaf comprising:
a spine with a free distal end;
a membrane connected to the respective spine so that the spine is fixed relative to the membrane;
a first flexible circuit disposed on the membrane on a first side of the spine;
a second flexible circuit disposed on the membrane on a second side of the respective spine, the second flexible circuit comprising a second electrode;
a first ablation electrode disposed on the first flexible circuit; and
a second ablation electrode disposed on either the first flexible circuit or the second flexible circuit; and
an ablation generator configured to provide ablation pulses between the first ablation electrode and the second ablation electrode of each leaf.

14. The medical system of claim 13, the second ablation electrode of each leaf being disposed on the second flexible circuit of the respective leaf, optionally each leaf further comprising a third ablation electrode disposed on the first flexible circuit and a fourth ablation electrode disposed on the second flexible circuit, the ablation generator being configured to provide ablation pulses between the third ablation electrode and the fourth ablation electrode of each leaf.

15. The medical system of claim 13, the second ablation electrode of each leaf being disposed on the first flexible circuit of the respective leaf, optionally each leaf further comprising a third ablation electrode disposed on the second flexible circuit and a fourth ablation electrode disposed on the second flexible circuit, the ablation generator being configured to provide ablation pulses between the third ablation electrode and the fourth ablation electrode of each leaf.

16. A method of manufacturing an end effector for a medical probe, the method comprising:
forming a plurality of spines each comprising a free distal end, the free distal ends extending away from a longitudinal axis;
disposing first flexible circuits on a first side of each spine of the plurality of spines, each first flexible circuit comprising a first flexible substrate and a first electrode;
disposing second flexible circuits on a second side of each spine of the plurality of spines, each second flexible circuit comprising a second flexible substrate and a second electrode;
placing a first sheet of insulative material in contact with the first flexible circuits;
placing a second sheet of insulative material in contact with the second flexible circuits;
molding the first sheet and the second sheet to envelop the first flexible circuits and the second flexible circuits; and
defining voids in the first and the second sheet such that the first sheet, the second sheet, the first flexible circuits, the second flexible circuits, and the spines collectively form a plurality of leaves, the leaves comprising free distal ends that are separated from one another.
